# EUROPEAN PATENT APPLICATION

(11) **EP 2 390 662 A1**
(43) Date of publication of application: **30.11.2011**
(21) Application number: 10164196.7
(22) Date of filing: 27.05.2010
(51) Int. Cl.: G01N 33/50, C12Q 1/68

(54) **Molecular classification of multiple myeloma**

(71) Applicant: Erasmus University Medical Center Rotterdam, 3015 GD Rotterdam (NL)
(72) Inventor: Sonneveld, Pieter, 3015 GE Rotterdam (NL)
(74) Representative: Habets, Winand

(57) **Abstract**

The present invention is in the field of molecular diagnostics and relates to a method for classifying samples obtained from patients diagnosed with multiple myeloma into three newly defined clusters. The invention also relates to a method for determining the prognosis of an individual diagnosed with multiple myeloma as well as a method for the prediction of the response to treatment of an individual diagnosed with multiple myeloma.

## Description

### Introduction

The present invention is in the field of molecular diagnostics and relates to a method for classifying samples obtained from patients diagnosed with multiple myeloma. The invention also relates to a method for determining the prognosis of an individual diagnosed with multiple myeloma as well as a method for the prediction of the response to treatment of an individual diagnosed with multiple myeloma.

### Background of the invention

Several research reports have already issued dealing with the determination of gene expression profiles based on RNA micro-array technology in patients with newly diagnosed MM.¹⁻⁶ Two major genetic classification systems have been developed, the Translocation and cyclin D (TC) classification and the UAMS (University of Arkansas for Medical Science) molecular classification of myeloma. The TC classification distinguishes eight subgroups based on over-expression of genes deregulated by primary IgH translocations and transcriptional activation of cyclin D genes.⁷ The UAMS molecular classification of myeloma identified seven tumor groups characterized by distinct gene expression profiles, including translocation clusters MS (t(4;14)); MF (t(14;16)/t(14;20)), and CD-1/2 (t(11;14) and t(6;14)), as well as a hyperdiploid cluster, HY, a cluster with proliferation-associated genes (PR) and a cluster without a clear signature but characterized by a low percentage of bone disease (LB)_{.}⁵

It is important to classify patients with an established diagnosis of multiple myeloma into distinct clusters because it has been found that patients in these clusters may have a different response to therapy and/or a better prognosis.

### Summary of the invention

Unsupervised hierarchical clustering of 320 samples obtained at diagnosis of multiple myeloma from primarily Caucasian, North-European subjects resulted in a subdivision in 10 clusters rather than seven or eight, as disclosed in the prior art.

The present invention provides three novel clusters which were found to be relevant for the classification of patients with multiple myeloma, i.e. clusters NFκB, CTA and PRL3.

In one aspect the present invention relates to a method for classifying a patient diagnosed with multiple myeloma into at least one of the clusters NFκB, CTA or PRL3, said method comprising the step of determining the expression level of certain genes in a sample isolated from said patient wherein said patient is classified into cluster NFκB if at least two genes are overexpressed selected from the group consisting of CDC42, TNFAIP3, BCL10. IL8, GADD45B, NFKBIE and MIRN155, or into cluster CTA if at least two genes are overexpressed selected from the group consisting of HIST1H2BG, GINS1, CDC2, MAGEA3, BIRC5, MAGEA6, MAGEA12, PAGE1, PTTG1 and GAGE, or into cluster PRL3 if at least two genes are overexpressed selected from the group consisting of CCND2, PRL3, PTPRZ1, SOCS3 and SMYD3.

The expression levels of the genes selected from the above groups are shown in tables 1 to 3.

The new clusters identified herein comprise patients with an improved prognosis and a distinct response to therapy. Therefore, the invention also relates to a method for determining the prognosis of a patient diagnosed with multiple myeloma by classifying said patient into at least one of clusters NFκB, CTA or PRL3, said method comprising the step of determining the expression level of certain genes in a sample isolated from said patient wherein said patient is classified into cluster NFκB if at least two genes are overexpressed selected from the group consisting of CDC42, TNFAIP3, BCL10. IL8, GADD45B, NFKBIE and MIRN155, or into cluster CTA if at least two genes are overexpressed selected from the group consisting of HIST1H2BG, GINS1, CDC2, MAGEA3, BIRC5, MAGEA6, MAGEA12, PAGE1, PTTG1 and GAGE, or into cluster PRL3 if at least two genes are overexpressed selected from the group consisting of CCND2, PRL3, PTPRZ1, SOCS3 and SMYD3.

Also, the invention relates to a method for determining the response to therapy by classifying a patient diagnosed with multiple myeloma into at least one of clusters NFκB, CTA or PRL3, said method comprising the step of determining the expression level of certain genes in a sample isolated from said patient wherein said patient is classified into cluster NFκB if at least two genes are overexpressed selected from the group consisting of CDC42, TNFAIP3, BCL10. IL8, GADD45B, NFKBIE and MIRN155, or into cluster CTA if at least two genes are overexpressed selected from the group consisting of HIST1H2BG, GINS1, CDC2, MAGEA3, BIRC5, MAGEA6, MAGEA12, PAGE1, PTTG1 and GAGE, or into cluster PRL3 if at least two genes are overexpressed selected from the group consisting of CCND2, PRL3, PTPRZ1, SOCS3 and SMYD3.

According to a particular embodiment of the present invention the sample from said patient comprises plasma cells.

According to a further embodiment of the present invention the sample from the patient comprises plasma cells selected for CD138 expression.

As used herein, the term overexpressed relates to the level of expression of a certain gene as expressed in the amount of RNA transcribed from that gene. Overexpressed genes produce more RNA than the corresponding gene in a normal subject. Preferably, overexpressed genes are genes expressing at least 2 times the amount of RNA as compared to a normal subject, preferably more than 3, such as 4, 5, 6, 7 10, 15 or even more than 20. See tables 1 to 3 for the expression levels of the genes identifying the clusters as described herein.

It was found that determining the expression of at least two genes selected from the groups as described above was sufficient for effective classification of a patient in one of the newly identified clusters. However, the methods provided even more reliable results when the expression levels of three or more genes selected from the groups as defined above were determined, such as 4 or 5 or 6 genes. Even more reliable results were obtained when the expression levels of all genes selected from the groups as defined above were determined.

The NFκB cluster was characterized by clear differential expression of genes involved in the NFκB pathway. Highly expressed NFκB genes include *BCL10, TNFAIP3, IL8, GADD45B and NFKNIE.* Many other genes were also overexpressed, however, they did not seem to be essential in the classification for the NFkB cluster. Highly expressed, non-essential genes within the NFkB cluster were for instance TNFIP1, NFKBIZ, IL2RG, CD40 and CD74.

NFκB signaling is crucial in the pathogenesis of myeloma^{8,9} involving both inactivating and activating mutations which primarily result in constitutive activation of the non-canonical NFκB pathway.^{8,9} Cases with high expression values of probe sets corresponding to NFκB genes CD74, IL2RG and TNFAIP3, show a better response to Bortezomib, however no change in progression free survival (PFS), whereas patients with low TRAF3 expression show a better response to Bortezomib and a prolonged PFS.⁹ The NFκB-index based on CD74, IL2RG and TNFAIP3 was significantly higher in our NFκB cluster. In keeping with this, negative regulators of NFκB signaling showed reduced expression, e.g. TRAF3, whereas genes involved in stimulating NFκB activity, e.g. CD40, were found to be increased.

The second distinct novel subgroup observed here is the CTA cluster. This resembles the PR cluster concerning the presence of poor prognostic markers such as CTA genes, the highest percentage of patients with an extreme high-risk index, and overexpression of *AURKA* and *BIRC5.* Although proliferation associated genes such as *AURKA* and *BIRC5* as well as cell cycle genes such as CDC2 and CDC42 were among the top overexpressed genes in the CTA cluster, the CTA cluster showed a significantly lower proliferation index (PI) in comparison to the PR cluster. An explanation could be the absence of a number of genes representing the Pl. Alternatively, the fold change difference of present genes between the CTA cluster and the remaining clusters was lower than the fold change difference in PR cluster vs. remaining clusters. Besides features such as a higher percentage of 1q gain and a significantly higher PI, no clinical features distinguished the CTA from the PR cluster.

Overexpression of at least two genes selected from the group consisting of CCND2, PRL3, PTPRZ1, SOCS3 and SMYD3 was indicative for the PRL3 cluster. Remarkably, protein tyrosine phosphatases *PRL3, PTPRZ1* and SOCS3 were among the overexpressed genes in this cluster. Higher PRL3 expression was found in bone marrow plasma cells from patients with newly diagnosed monoclonal gammopathies than in plasma cells from healthy donors and significantly higher in the UAMS PR, LB and MS groups. Silencing of PRL3 by siRNA impaired SDF-1-induced migration of MM cells, but no influence on cell-cycle distribution or cell proliferation was observed.¹¹

PTPRZ1 is involved in the regulation of protein phosphorylation and plays a critical function in signal transduction, cell growth, differentiation and oncogenesis.^{12,13}

SOCS3 is a cytokine-inducible negative regulator of cytokine signaling. The expression of this gene is induced by various cytokines, including IL6, IL10, and interferon (IFN)-gamma. Transfection of myeloma cell lines with SOCS3 showed protection from growth suppression by IFN-alpha. IL6 induced by IFN-alpha may play an important role in the growth and survival of myeloma cells, and upregulated SOCS3 by IL6 may be at least partially responsible for the IL6-mediated inhibition of IFN-alpha signaling in myeloma cells.¹⁴⁻¹⁶

PRL-3 overexpression in mammalian cells was reported to inhibit angiotensin-II induced cell calcium mobilization and promote cell growth. Absence of poor prognostic factors such as 17p loss, combined with low values for high-risk index, proliferation index and AURKA expression suggests cases within this cluster may have less severe disease *(p≤0.001).* Indeed, the frequency of ISS I was markedly higher in this cluster than in the other clusters.

A comparison to existing classifications confirmed the 7 clusters described in the UAMS classification, CD-1, CD-2, MS, MF, HY, PR and LB.⁵ Furthermore, Zhan et al. reported a group of cases with a myeloid signature which was excluded from further analyses.⁵ The patients in this so-called Contaminated cluster showed less disease activity and performed better on treatment, with significantly prolonged EFS and OS. We retained the group of patients with a myeloid signature in the gene expression analysis. These samples clustered together, clinically characterized by a significantly lower level of bone marrow plasmacytosis. Classifiers were build to validate the clusters. High sensitivity (Sn) and specificity (Sp) values were found for the classifiers of clusters CD-2, MS, MF and HY with Sn varying from 84% to 97% and Sp from 91% to 100%. Lower Sn was observed with classifiers for clusters CD-1 and PR. The classifier for the Myeloid cluster consisting of 87 probe sets yielded the lowest Sn and Sp. The CTA, NFκB and PRL3 cluster were novel clusters and could therefore not be validated using the UAMS cluster definitions.

Furthermore, we validated our classification by applying our sample and gene selection criteria to 538 UAMS raw data files representing newly diagnosed MM cases and 264 APEX/SUMMIT/CREST raw data files representing relapsed MM cases. Sample clustering resulted in confirmation of seven clusters: CD1, CD2, MS, MF, HY, PR and Myeloid clusters in both datasets. In addition, in both sets we observed a combined NFκB / LB cluster, showing overexpression of genes involved in the NFκB signaling pathway, but also of *PHACTR3, RASGRP1, IL6R* and downstream targets of MAF/MAFB. In our clustering, LB samples were found as a subcluster of the MF cluster, based on expression of MAFB and c-MAF downstream targets. This LB subcluster might represent a subgroup corresponding to an earlier stage of disease, as suggested by the lack of poor prognostic markers, such as thrombocytopenia and elevated LDH. The HOVON65/GMMG-HD4 NFκB cluster and LB subcluster were observed as two distinct clusters; except for a high NFκB-index, no overlap in differentially expressed genes, or percentage of bone lesions was observed. Merging of these two clusters in independent datasets might be possible based on a weaker expression of NFκB related genes showing lower fold changes relative to LB cluster genes and MAF/MAFB downstream targets. However the presence of a cluster mainly characterized by an NFκB-index cannot be disputed. We also confirmed the PRL3 cluster based on the overexpression of at least PRL3 among the top 10 genes showing the highest fold change difference, and SOCS3, CCND2 and/ or PTPRZ1. A CTA like cluster was found in the APEX dataset characterized by a different CTA expression profile compared to our CTA cluster. No CTA cluster was detected in the UAMS dataset; samples with a CTA signature clustered within the PR, HY, MS and Myeloid cluster.

In the UAMS classification the PR, MS and MF clusters were defined as high-risk groups with a significantly lower PFS and OS.⁵ In agreement to this report, we demonstrated associations between clusters PR, MS, MF, the novel cluster CTA and poor prognostic factors, such as increased high-risk index and elevated LDH.

**Table 1**

| First column, genes significant at the nominal 0.001 level of the univariate test within a false discovery rate (FDR) of 5%; second column, geometric mean of intensities per probe set in class 1; third column, geometric mean of intensities per probe set in class 2; fourth column, fold change, which is the factor difference between the geometric mean of a probe set of class 1 versus class 2, fifth column, probe set ID; sixth column, description of the probe set; seventh column, gene symbol. | | | | | | |
|---|---|---|---|---|---|---|
| Cluster CTA | | | | | | |
| **FDR** | **Geom mean of intensities in class 1** | **Geom mean of intensities in class 2** | **Fold change** | **Probe set** | **Description** | **Gene symbol** |
| < 1e-07 | 6,599521 | 0,8699596 | 7,5860086 | 210387_at | histone cluster 1, H2bg | HIST1H2BG |
| < 1e-07 | 4,485077 | 0,8951231 | 5,01057 | 206102_at | GINS complex subunit 1 (Psf1 homolog) | GINS1 |
| 3,49E-05 | 3,8199491 | 0,9057937 | 4,2172395 | 203213_at | cell division cycle 2, G1 to S and G2 to M | CDC2 |
| 0,003504 | 3,6945174 | 0,9080291 | 4,0687215 | 209942_x_at | melanoma antigen family A, 3 | MAGEA3 |
| < 1e-07 | 3,6358618 | 0,9091026 | 3,9993966 | 202095_s_at | baculoviral IAP repeat-containing 5 (survivin) | BIRC5 |
| 0,004231 | 3,3310771 | 0,9149975 | 3,6405312 | 214612_x_at | melanoma antigen family A, 6 | MAGEA6 |
| < 1e-07 | 2,9665801 | 0,9228592 | 3,2145532 | 210467_x_at | melanoma antigen family A, 12 | MAGEA12 |
| 1,26E-05 | 2,8862304 | 0,9247319 | 3,1211537 | 206897_at | P antigen family, member 1 (prostate associated) | PAGE1 |
| 1,09E-05 | 2,8637685 | 0,9252654 | 3,0950779 | 203554_x_at | pituitary tumor-transforming 1 | PTTG1 |
| 3,35E-05 | 2,7283461 | 0,9285804 | 2,9381906 | 208235_x_at | G antigen | GAGE |

**Table 2**

| First column, genes significant at the nominal 0.001 level of the univariate test within a false discovery rate (FDR) of 5%; second column, geometric mean of intensities per probe set in class 1; third column, geometric mean of intensities per probe set in class 2; fourth column, fold change, which is the factor difference between the geometric mean of a probe set of class 1 versus class 2, fifth column, probe set ID; sixth column, description of the probe set; seventh column, gene symbol. | | | | | | |
|---|---|---|---|---|---|---|
| Cluster NFkB | | | | | | |
| **FDR** | **Geom mean of intensities in class 1** | **Geom mean of intensities in class 2** | **Fold change** | **Probe set** | **Description** | **Gene symbol** |
| < 1e-07 | 13,6270564 | 0,7106994 | 19,174149 | 214230_at | cell division cycle 42 (GTP binding protein, 25kDa) | CDC42 |
| < 1e-07 | 8,8220653 | 0,7522708 | 11,727247 | 202643_s_at | tumor necrosis factor, alpha-induced protein 3 | TNFAIP3 |
| < 1e-07 | 7,7958985 | 0,7645318 | 10,196957 | 1557257_at | B-cell CLL/lymphoma 10 | BCL10 |
| < 1e-07 | 5,5999541 | 0,798327 | 7,0146118 | 202859_x_at | interleukin 8 | IL8 |
| < 1e-07 | 4,9789671 | 0,8106896 | 6,1416444 | 209305_s_at | growth arrest and DNA-damage-inducible, beta | GADD45B |
| < 1e-07 | 4,9564081 | 0,811171 | 6,1101888 | 203927_at | nuclear factor of kappa light polypeptide gene enhancer in B-cells inhibitor, epsilon | NFKBIE |
| 0,000169 | 3,2049201 | 0,8587533 | 3,7320615 | 229437_at | microRNA 155 | MIRN155 |

**Table 3**

| First column, genes significant at the nominal 0.001 level of the univariate test within a false discovery rate (FDR) of 5%; second column, geometric mean of intensities per probe set in class 1; third column, geometric mean of intensities per probe set in class 2; fourth column, fold change, which is the factor difference between the geometric mean of a probe set of class 1 versus class 2, fifth column, probe set ID; sixth column, description of the probe set; seventh column, gene symbol. | | | | | | |
|---|---|---|---|---|---|---|
| Cluster PRL3 | | | | | | |
| **FDR** | **Geom mean of intensities in class 1** | **Geom mean of intensities in class 2** | **Fold change** | **Probe set** | **Description** | **Gene symbol** |
| 0,040142 | 17,7417336 | 0,9201432 | 19,281492 | 200953_s_at | cyclin D2 | CCND2 |
| 0,007963 | 14,4896027 | 0,9255509 | 15,655113 | 206574_s_at | protein tyrosine phosphatase type IVA, member 3 /// similar to protein tyrosine phosphatase type IVA, member 3 | PRL3 |
| < 1e-07 | 9,1313678 | 0,9380007 | 9,7349268 | 204469_at | protein tyrosine phosphatase, receptor-type, Z polypeptide 1 | PTPRZ1 |
| < 1e-07 | 5,6432704 | 0,9511555 | 5,9330682 | 227697_at | suppressor of cytokine signaling 3 | SOCS3 |
| 0,025799 | 5,6056028 | 0,9513398 | 5,8923243 | 217865_at | ring finger protein 130 | RNF130 |
| 0,00099 | 3,9471646 | 0,961046 | 4,1071548 | 218788_s_at | SET and MYND domain containing 3 | SMYD3 |

### Examples

### Example 1

### Cell isolation and analysis

Plasma cell (PC) purification of bone marrow samples from included patients was performed in 11 centers equipped to perform PC purification across the Netherlands, Germany and Belgium. PCs were separated using positive magnetic cell sorting (MACS) selection with CD138 magnetic microbeads (Miltenyi Biotec B.V., Utrecht, The Netherlands). Next, purified samples were analysed for purity and viability by flow cytometric analysis (FACS Calibur and Cell Quest software, BD biosciences, Alphen a.d. Rijn, the Netherlands) with CD138-PE (Beckman Coulter, Mijdrecht, NL), annexin-FITC (NeXins Research, Kattendijke, The Netherlands) and 7-AAD (Beckman Coulter, Mijdrecht, NL). Protocols for PC purification and FACS analysis were equal in all centers. Purified PCs were stored in RLT buffer at -80 until collection.

### Example 2

### Preparation of cRNA

RNA isolation was performed in Erasmus Medical Center and at the University of Heidelberg. Only samples with a monoclonal PC purity > 80% were used for analysis. RNA was isolated from purified PCs using a DNA/RNA prep kit (Qiagen, Venlo, The Netherlands). RNA concentration was measured using the NanoDrop spectrophotometer (Thermo Fisher Scientific, Landsmeer, The Netherlands). RNA quality and purity was assessed using the RNA 6000 pico or nano assay (Agilent 2100 Bioanalyzer, Agilent Technologies Netherlands B.V., Amstelveen, The Netherlands).

### Example 3

### Gene expression profiling and array analysis

RNA processing, target labeling and hybridization to gene expression arrays was performed exclusively in the Erasmus Medical Center.

Biotin labeled cRNA was obtained using the Two-Cycle Eukaryotic Target Labeling Assay (Affymetrix). 15 µg of fragmented, biotin labeled cRNA was hybridized to AffymetrixⓇ GeneChip® Human Genome U133 plus 2.0 arrays according to standard Affymetrix protocol (Affymetrix Inc, Santa Clara, CA)

Quality controls of arrays using GeneChip® Operating Software (GCOS) included scaling factor (SF) and percentage of genes present (GP). Arrays with SF difference < 3 and GP > 20% were analyzed further. Raw data from selected gene expression arrays (CEL-files) were pre-processed using GCRMA in Partek Genomics Suite, version 6.4 (Partek®, St. Louis, MO, USA). Final quality control of arrays included relative log expression (RLE) and normalized unscaled standard errors (NUSE) from the AffyPLM package (www.bioconductor.com). Arrays showing a NUSE value > 1.05 and aberrant RLE plots were excluded from analysis. Microarray data presented in this paper can be retrieved from the NIH Gene Expression Omnibus (GEO; National Center for Biotechnology Information [NCBI], accession number GSE19784.

### Example 4

### Cluster analysis

GCRMA normalized expression data were imported in Omniviz software version 6.0.1.(Biowisdom, Harston, Cambridge, UK) In Omniviz, the exponential values were taken of the GCRMA derived log2 intensity values, and as GeneChips do not reliably discriminate between values below 30, all intensity values below 30 were set to 30. The level of expression for every probe set was determined relative to the geometric mean (GM) and log transformed (base 2). The 5% (2730) most variable probe sets from the total were selected using a cut-off of log2GM < -5.12 or > 5.12 (reflecting up- or down-regulation) in at least one patient. Hierarchical clustering of average linkage with the centered correlation metric was performed using BRB-array tools version 3.6.0 (http://linus.nci.nih.gov/BRB-ArrayTools.html). The dendrogram obtained was compared to translocation status, and robustness(R)-indices (BRB-array tools) were calculated to give an indication about reproducibility of the clusters. To determine expression signature of clusters, each cluster was compared to the remaining clusters using the Class Comparison option with the following settings: p<0.001, false discovery rate (FDR) <5% (BRB-array tools).

### Example 5

### Prediction analysis of micro-arrays

To validate clusters, a method of nearest shrunken centroid classification using prediction analysis of micro- arrays (PAM) in R version 2.6.0 (PAMr package in R version 2.6.0) was used.¹⁷ Validation of clusters was performed in an independent dataset, GSE2658, generated by the University of Arkansas for Medical Sciences which included 559 newly diagnosed MM patients. The dataset containing 5% most variable genes, 2730 genes, was used. Sensitivity (Sn), specificity (Sp), positive predictive values (PPV) and negative predictive values (NPV) were calculated.

In addition, validation analysis to confirm all identified clusters was performed using the CEL files of 2 independent datasets, the APEX/SUMMIT/CREST dataset,¹⁸ and the UAMS dataset.⁵ CEL files were normalized using our normalization methods, sample and gene selection criteria as described.

For the prediction analysis of translocations t(4;14), t(11;14) and t(14;16)/t(14;20), samples with available FISH data were randomly divided in a training set (2/3), and a test set (1/3). Training set and test set were separately normalized. For the training set, 5% most variable genes, 2730 genes, were generated using the method described above. For the test set the 2730 probe sets thus generated were used. Percentage correctly classified samples, Sn, Sp, PPV and NPV were calculated

### Example 6

### Cytogenetic analysis and FISH

FISH analysis was performed in 304 patients. In addition karyotyping data were available for 119 patients. In non-purified PC samples (n=125) at least 200 interphase nuclei per sample were analyzed using epi-fluorescence microscopy and image analysis software with in several cases a preceding analysis of selected myeloma cells based on light chain counterstaining or morphology. In CD138 purified PC samples (n=179) 100 nuclei were evaluated using an epi-fluorescence microscope (LEICA, Wetzlar, Germany). Hybridization efficiency was validated on PCs obtained from bone marrow of a healthy donor and thresholds for gains, deletions and translocations were set at 10%.

Interphase-FISH analysis was performed as previously described.^{19,20} Detection of numerical changes was performed using commercial 2-color probes chromosome loci 1q21/8p21, 11q23/13q14, 9q34/22q11, 6q21/15q22, and17p13/19q13 (Poseidon Probes, Kreatech, Amsterdam, Netherlands), or using Alpha satellite probes for centromere regions of chromosome 9 and 11 (CEP 9 and CEP 11) (Vysis, Abbott Molecular, Abbott Park, Illinois, USA). The combination of trisomies # 9, 11, and 15 was found to be predictive of hyperdiploidy.²¹ Hyperdiploid MM was defined by presence of trisomy of two of these chromosomes (trisomy 9 and 11, 11 and 15 or 9 and 15) or all of them (trisomy of chromosomes 9, 11 and 15), determined by FISH and/or karyotyping data.

Translocations t(11;14)(q13;q32), t(4;14)(p16;q32) and (14;16)(q32;q23) were determined using probes LSIIGH/CCND1, LSI IGH/FGFR3 and LSI IGH/MAF, respectively (Vysis, Abbott Molecular, Abbott Park, Illinois, USA) or commercial 2-color probe sets for the detection of translocations t(11 ;14)(q13;q32), t(4;14)(p16;q32) (both Poseidon Probes, Kreatech, Amsterdam, Netherlands) and t(14;16) (q32;q23) (Vysis, Downers Grove, IL). A t(14;20) (q32;q12) with 14q32 IGH gene rearrangement was confirmed by FISH using 14q32 IgH rearrangement probe, LSI IGH DC and whole chromosome paint 14 and 20 probes, wcp14 and wcp 20 (Vysis, Abbott Molecular, Abbott Park, Illinois, USA). Conventional karyotyping was performed as described before_{.}²²

### Example 7

### Identification of expression signatures

320 bone marrow aspirates from newly diagnosed patients were obtained upon inclusion in the HOVON65/GMMG-HD4 trial for gene expression profiling. Comparison of baseline clinical characteristics of this subset of patients showed no significant difference between characterized subset and the whole patient group in the trial.

Translocation status and robustness(R)-indices per cluster were determined. Of the eleven clusters found, 10 were characterized in detail. The remaining cluster, consisting of 9 samples with 41 differentially expressed genes was excluded from analysis, since no clear signature could be determined. Six of the identified clusters corresponded well to the published UAMS classification and were therefore named accordingly.⁵

Samples harboring t(11;14) and/or overexpression of CCND1 were divided into two clusters, CD-1 and CD-2. A relatively low frequency of t(11;14) (33%) was found in the CD-1 cluster in our study, which is low compared to previous reports.⁵ Still, this cluster was characterized by high CCND1 expression and by overexpression of argininosuccinate synthetase 1 *ASS1,* inhibin beta E *INHBE,* and nidogen 2 *NID2* as has been described before. B-cell markers *MS4A1 (CD20), VPREB3, CD79A* and *BANK1* defined cluster CD-2. CD20 expression has been associated with presence of t(11;14),²³ which is consistent with the high percentage of t(11;14) observed in this cluster in comparison to cluster CD-1.

The MS cluster was characterized by translocation t(4;14), deregulating *FGFR3 and MMSET,* present in 96% of patients in this cluster. Other notable overexpressed genes include desmoglein *DSG2, CCND2,* selectin L (lymphocyte adhesion molecule 1) *SELL,* and serpin peptidase inhibitors SERPINE2, SERPINI1. This cluster showed a significantly higher percentage of patients with a 1q21 amplification (61 %, compared to 8% to 50% in the remaining clusters (*p<0.001*)).

The MF cluster contained 32 samples of which 7 samples harbored a confirmed t(14;16) or t(14;20). c-MAF which is deregulated by t(14;16), and MAFB, deregulated by t(14;20), were observed only in a subset of patients, which clustered separately within this MF cluster. The remaining samples in the MF cluster clustered with these samples based on overexpression of downstream targets of *MAFB* and/or *c-MAF: RND3, CCND2,* and *ITGB7.*²⁴ *FRZB* and *DKK1,* both WNT inhibitors of which the presence is associated with osteolytic lesions in myeloma patients, were among the top downregulated genes.^{25,26} Analyzing both subsets separately revealed an even stronger signature for the MF subcluster. Clinical features such as elevated LDH and thrombocytopenia were predominantly present in the MF subcluster and significantly higher in comparison to the remaining clusters, 47% vs. 0% - 46% *(p=0.01)* and 35% vs. 0% - 21% *(p<0.001).* The remaining subset of 15 samples lacking translocations and clustering together only based on downstream targets, showed a gene signature with the top overexpressing genes corresponding to those overexpressed in the UAMS LB cluster, *RASGRP1* and *PHACTR3.* The MF cluster showed the lowest percentage of patients with bone lesions, 52% vs. 62%-100% in the remaining clusters *(p=0.004).* This percentage was even lower in the LB subcluster, 50% vs. 53%-100% *(p=0.04).*

Six clusters were characterized by high frequencies of hyperdiploidy, ranging from 57% to 94%. One of these clusters showed upregulation of erythroid and myeloid markers as well as genes involved in cell mediated immune response, humoral immune response, and antigen presentation. This cluster was indicated as the Myeloid cluster. No distinct clinical features characterized this cluster, as was observed in the UAMS classification regarding the low percentage of patients having IgA subtype, β2M and renal injury. However bone marrow plasma cell percentage before and after plasma cell purification was significantly lower in this cluster in comparison to remaining clusters, 30% vs. 50% *(p=0.008)* and 87% vs. 91% *(p<0.001),* respectively. The lower level of bone marrow plasmacytosis at diagnosis was also observed in the UAMS myeloid cluster.

The HY cluster showed hyperdiploidy in 94% of cases. This group was characterized by upregulation of death receptor *TNFSF10 (TRAIL),* interferon induced genes such as *IFIT1, IFIT3 and IF127,* WNT antagonists *FRZB* and *DKK1,* glucosidase, beta, acid 3 (cytosolic) *GBA3,* and *MYC* proto-oncogene.

Two predominantly hyperdiploid clusters showed upregulation of cancer testis antigens (CTA). These include *MAGEA3, MAGEA6F, MAGEA12, PAGE1 and GAGE12F.* Presence calls of some CTA genes have been reported to correlate with significantly shorter event free survival, i.e. *CTAG1B, CTAG2, MAGEA1, MAGEA2, MAGEA3,* and *MAGEA6.*²⁷ The latter two were among the top 50 upregulated genes in both clusters. In addition, cases with the 15% highest values of the high-risk index were predominantly observed in these clusters *(p<0.001).* The high-risk index is based on the published 17 gene model, which has been linked to early disease-related death.²⁸ The difference between these two clusters was based on overexpression of genes involved in cell cycle and proliferation in one of the clusters, with a significantly higher proliferation index (PI), based on the calculated median expression of 11 genes associated with proliferation: *TOP2A, BIRC5, CCNB2, NEK2, ANAPC7, STK6, BUB1, CDC2, C10orf3, ASPM,* and *CDCA1 (p<0.001).²⁹* This cluster was named PR cluster, described before by Zhan et al.⁵ The other CTA overexpressing cluster was mainly characterized by CTA genes and therefore named CTA cluster. Overlapping characteristics between the CTA and PR cluster were the overexpression of Aurora kinase A (*AURKA*), recently reported to be associated with a higher proliferation rate and poor outcome, which was significantly higher in both clusters in comparison to the remaining clusters *(p<0.001),* and even higher in the PR compared to the CTA cluster *(p=0.2)*.^{30,31} Also *BIRC5 (Survivin),* another recently described gene of which the presence call has been associated with lower EFS and OS in newly diagnosed multiple myeloma patients, was observed among the top 50 upregulated genes in PR and CTA cluster.³² The CTA cluster has not been described as a distinct entity before and is therefore proposed as a new cluster.

The second new cluster was characterized by clear differential expression of genes involved in the NFκB pathway. Highly expressed NFκB genes include *BCL10, TNFAIP3, IL8, GADD45B, NFKNIE, TNFIP1, NFKBIZ, IL2RG, CD40* and *CD74.* In addition, the NFκB-index as reported by Keats et al., based on the mean expression level of four probe sets corresponding to *CD74, IL2RG,* and *TNFAIP3* (2×), as well as the NFκB-index published by Annunziata et al., based on the mean expression of 11 probe sets *(BIRC3, TNFAIP3, NFK82, IL2RG, NFKBIE, RELB, NFKBIA, CD74, PLEK, MALT1, WNT10A)* were significantly higher in this cluster compared to the other clusters *(p<0.001)*.^{8,9}

Based on these characteristics this cluster was termed NFκB cluster. Important regulators of the NFκB pathway were further analyzed. *CD40* and *NIK* (NFκB-inducing kinase) expression are both involved in activation of the NFκB pathway. Only CD40 expression was significantly higher *(p<0.001),* whereas the TNF-receptor-associated factor 3 *TRAF3,* a negative NFκB regulator showed significantly lower expression in the NFκB cluster *(p=0.004).*

The third new cluster consisted of 9 cases and only 27 genes were differentially expressed, including overexpression of protein tyrosine phosphatase *PTP4A3 (PRL-3),* protein tyrosine phosphatase, receptor-type, Z polypeptide 1 *PTPRZ1* and suppressor of cytokine signaling 3 SOCS3. In lieu of any other characteristic this cluster was termed PRL3 cluster. Chromosomal characteristics include hyperdiploidy in 75% of patients in this cluster, 1q gain was observed in 38% of patients; however no 17p loss was observed. Strikingly, all patients in this cluster exhibited bone lesions. Furthermore this cluster had the highest percentage of patients in ISS stage I, 67% vs. 19%-57% in remaining clusters *(p=0.062).*

Expression levels of certain important genes in different clusters (*MMSET, FGFR3, CCND1, INHBE, ASS1, VPREB3, MS4A1, NUAK1* and *RND3* were successfully verified by quantitative RT-PCR.

### Example 8

### A classifier for validation of clusters

To validate clusters described here, we used the dataset upon which the UAMS classification is based (GSE2658). We performed PAM analysis of corresponding clusters using the UAMS cluster definitions.⁵ High sensitivity (Sn) and specificity (Sp) values were found for the classifiers of clusters CD-2, MS, MF and HY with Sn varying from 84% to 97% and Sp from 91 % to 100%. Lower Sn was observed with classifiers for clusters CD-1 and PR. The classifier for the Myeloid cluster consisting of 87 probe sets yielded the lowest Sn and Sp. The CTA, NFκB and PRL3 cluster were novel clusters and could therefore not be validated using the UAMS cluster definitions. In addition, our clustering was compared to the TC classification,² and to the UAMS classification.⁵ To this end TC criteria were used to assign the samples to the TC classes and the published top 50 up and top 50 downregulated probe sets which defined the 7 UAMS clusters to cluster our dataset. The MF subcluster, as defined above, corresponded well to the Maf TC class; the MS cluster corresponded well to the 4p16 TC class. Samples from our CD-1/2 clusters corresponded to 11q13 and D1 classes.

Due to the limited nature of the TC-classification, the classes did not compare to any of our other clusters. Regarding the UAMS classification we confirmed the 7 described clusters. In addition we identified a cluster showing a high NFκB-index and overexpression of *BCL10,* which was observed among the top upregulated genes in our NFκB cluster. No other genes involved in the NFκB pathway were present in the used gene set. Furthermore we observed that HOVON65/GMMG-HD4 samples originally present in the NFκB cluster were now shifted to this extra cluster, which therefore probably represents the NFκB cluster.

For additional validation of our classification including the novel described clusters we used 2 independent datasets, i.e. the UAMS data and a separate set of data from relapsed MM cases included in the APEX/SUMMIT/CREST trials to which we applied our normalization methods and gene selection criteria.¹⁸

From the UAMS data set 548 CEL files were made available. After performing quality control using NUSE 10 arrays were excluded. The 2730 most variable genes of the remaining 538 samples were selected as described. 1255 genes overlapped with the HOVON65/GMMG-HD4 gene set. Clustering resulted in the identification of the translocation clusters, HY, PR and myeloid cluster. We identified an NFκB cluster with upregulation of genes involved in the NFκB pathway such as *TNFAIP3, CFLAR, NFK82, PLEK, IL2RG* and *CD74* and a high NFκB-index, and additionally genes upregulated in the UAMS LB cluster such as *PHACTR3, RASGRP1, lL6R, BIK* and *EDN1.* This cluster clustered next to the MF cluster with subsequent upregulation of *RND3, AHNAK, CCND2* and *ARL4C.* Downregulated genes included *CCR2, TNFSF10, DKK1, FRZB* and interferon-induced genes. This cluster consists of UAMS LB and contaminated samples. Furthermore we identified a PRL3 cluster based on overexpression of *PRL3* and SOCS3. No separate CTA cluster was identified. Based on the 100 up/downregulated genes characterizing the CTA cluster, we observed that 7% samples (n=37) with highest/lowest expression of these genes were found mainly within the UAMS PR cluster (n=15), MS (n=5), HY (n=5) and contaminated cluster (n=5).

The APEX/SUMMIT/CREST data set consisted of 264 gene expression profiles of relapsed MM patients; all of the U133A and B arrays used showed good NUSE values. Gene selection by the criteria used in the present study yielded 2248 probe sets. The overlap with HOVON65/GMMG-HD4 gene set was 1002 genes. Again the translocation clusters, HY, PR and myeloid cluster were identified. In addition we detected an NFκB cluster with upregulation of NFκB related genes such as *TNFAIP3, IL2RG, CFLAR, NFKBIA, LMNA* and *KLF6,* but also genes upregulated in the UAMS LB cluster such as *PHACTR3, RASGRP1* and *IL6R* and genes frequently upregulated in the MF cluster such as *AHNAK, CCND2* and *ARL4C.* Furthermore, we identified a PRL3 cluster based on overexpression of *CCND2, PRL3, PTPRZ1* and a CTA like cluster. The CTA like cluster was defined by a different CTA profile than observed in the CTA cluster in our dataset, with upregulation of *SSX3, SSX4B,* and *MAGE2B.*

### Example 9

### A classifier for translocations

Samples with available FISH data were used to develop class predictors for translocations. For translocation t(11;14) the lowest classification error generated a classifier of only 5 probe sets among which multiple probe sets of CCND1 and KCNMB2, yielding Sn of 83% and Sp of 97%. For translocation t(4;14) a 25 probe set classifier generated Sn of 100% and Sp of 97%. Since samples with t(14;16) and t(14;20) clustered together, a combined t(14;16)/ t(14;20) classifier of 18 probe sets was generated which yielded Sn of 100% and Sp of 99%.

### References

1. Agnelli L, Bicciato S, Fabris S, et al. Integrative genomic analysis reveals distinct transcriptional and genetic features associated with chromosome 13 deletion in multiple myeloma. Haematologica. 2007;92:56-65.
2. Bergsagel PL, Kuehl WM. Molecular pathogenesis and a consequent classification of multiple myeloma. J Clin Oncol. 2005;23:6333-6338.
3. Decaux O, Lode L, Magrangeas F, et al. Prediction of survival in multiple myeloma based on gene expression profiles reveals cell cycle and chromosomal instability signatures in high-risk patients and hyperdiploid signatures in low-risk patients: a study of the Intergroupe Francophone du Myelome. J Clin Oncol. 2008;26:4798-4805.
4. Moreaux J, Hose D, Reme T, et al. CD200 is a new prognostic factor in multiple myeloma. Blood. 2006;108:4194-4197.
5. Zhan F, Huang Y, Colla S, et al. The molecular classification of multiple myeloma. Blood. 2006;108:2020-2028.
6. Chng WJ, Kumar S, Vanwier S, et al. Molecular dissection of hyperdiploid multiple myeloma by gene expression profiling. Cancer Res. 2007;67:2982-2989.
7. Bergsagel PL, Kuehl WM, Zhan F, Sawyer J, Barlogie B, Shaughnessy J, Jr. Cyclin D dysregulation: an early and unifying pathogenic event in multiple myeloma. Blood. 2005;106:296-303.
8. Annunziata CM, Davis RE, Demchenko Y, et al. Frequent engagement of the classical and alternative NF-kappaB pathways by diverse genetic abnormalities in multiple myeloma. Cancer Cell. 2007;12:115-130.
9. Keats JJ, Fonseca R, Chesi M, et al. Promiscuous mutations activate the noncanonical NF-kappaB pathway in multiple myeloma. Cancer Cell. 2007;12:131-144.
10. Jono H, Lim JH, Chen LF, et al. NF-kappaB is essential for induction of CYLD, the negative regulator of NF-kappaB: evidence for a novel inducible autoregulatory feedback pathway. J Biol Chem. 2004;279:36171-36174.
11. Fagerli UM, Holt RU, Holien T, et al. Overexpression and involvement in migration by the metastasis-associated phosphatase PRL-3 in human myeloma cells. Blood. 2008;111:806-815.
12. Hunter T. Protein kinases and phosphatases: the yin and yang of protein phosphorylation and signaling. Cell. 1995;80:225-236.
13. Sun H, Tonks NK. The coordinated action of protein tyrosine phosphatases and kinases in cell signaling. Trends Biochem Sci. 1994;19:480-485.
14. Catlett-Falcone R, Landowski TH, Oshiro MM, et al. Constitutive activation of Stat3 signaling confers resistance to apoptosis in human U266 myeloma cells. Immunity. 1999;10:105-115.
15. Thyrell L, Hjortsberg L, Arulampalam V, et al. Interferon alpha-induced apoptosis in tumor cells is mediated through the phosphoinositide 3-kinase/mammalian target of rapamycin signaling pathway. J Biol Chem. 2004;279:24152-24162.
16. Usui E, Nishii K, Katayama N, et al. Upregulated production of IL-6, but not IL-10, by interferon-alpha induces SOCS3 expression and attenuates STAT1 phosphorylation in myeloma cells. Hematol J. 2004;5:505-512.
17. Tibshirani R, Hastie T, Narasimhan B, Chu G. Diagnosis of multiple cancer types by shrunken centroids of gene expression. Proc Natl Acad Sci U S A. 2002;99:6567-6572.
18. Mulligan G, Mitsiades C, Bryant B, et al. Gene expression profiling and correlation with outcome in clinical trials of the proteasome inhibitor bortezomib. Blood. 2007;109:3177-3188.
19. Cremer FW, Bila J, Buck I, et al. Delineation of distinct subgroups of multiple myeloma and a model for clonal evolution based on interphase cytogenetics. Genes Chromosomes Cancer. 2005;44:194-203.
20. van Zutven LJ, Velthuizen SC, Wolvers-Tettero lL, et al. Two dual-color split signal fluorescence in situ hybridization assays to detect t(5;14) involving HOX11 L2 or CSX in T-cell acute lymphoblastic leukemia. Haematologica. 2004;89:671-678.
21. Chng WJ, Van Wier SA, Ahmann GJ, et al. A validated FISH trisomy index demonstrates the hyperdiploid and nonhyperdiploid dichotomy in MGUS. Blood. 2005;106:2156-2161.
22. Segeren CM, Sonneveld P, van der Holt B, et al. Overall and event-free survival are not improved by the use of myeloablative therapy following intensified chemotherapy in previously untreated patients with multiple myeloma: a prospective randomized phase 3 study. Blood. 2003;101:2144-2151.
23. Robillard N, Avet-Loiseau H, Garand R, et al. CD20 is associated with a small mature plasma cell morphology and t(11;14) in multiple myeloma. Blood. 2003;102:1070-1071.
24. van Stralen E, van de Wetering M, Agnelli L, Neri A, Clevers HC, Bast BJ. Identification of primary MAFB target genes in multiple myeloma. Exp Hematol. 2009;37:78-86.
25. Giuliani N, Morandi F, Tagliaferri S, et al. Production of Wnt inhibitors by myeloma cells: potential effects on canonical Wnt pathway in the bone microenvironment. Cancer Res. 2007;67:7665-7674.
26. Tian E, Zhan F, Walker R, et al. The role of the Wnt-signaling antagonist DKK1 in the development of osteolytic lesions in multiple myeloma. N Engl J Med. 2003;349:2483-2494.
27. Condomines M, Hose D, Raynaud P, et al. Cancer/testis genes in multiple myeloma: expression patterns and prognosis value determined by microarray analysis. J Immunol. 2007;178:3307-3315.
28. Shaughnessy JD, Jr., Zhan F, Burington BE, et al. A validated gene expression model of high-risk multiple myeloma is defined by deregulated expression of genes mapping to chromosome 1. Blood. 2007;109:2276-2284.
29. Perou CM, Jeffrey SS, van de Rijn M, et al. Distinctive gene expression patterns in human mammary epithelial cells and breast cancers. Proc Natl Acad Sci USA. 1999;96:9212-9217.
30. Hose D, Reme T, Meissner T, et al. Inhibition of aurora kinases for tailored risk-adapted treatment of multiple myeloma. Blood. 2009;113:4331-4340.
31. Chng WJ, Braggio E, Mulligan G, et al. The centrosome index is a powerful prognostic marker in myeloma and identifies a cohort of patients that might benefit from aurora kinase inhibition. Blood. 2008;111:1603-1609.
32. Jourdan M, Reme T, Goldschmidt H, et al. Gene expression of anti- and pro-apoptotic proteins in malignant and normal plasma cells. Br J Haematol. 2009;145:45-58.

## Claims

1. A method for classifying a patient diagnosed with multiple myeloma into at least one of the clusters NFκB, CTA or PRL3, said method comprising the step of determining the expression level of certain genes in a sample isolated from said patient wherein said patient is classified into cluster NFκB if at least two genes are overexpressed selected from the group consisting of CDC42, TNFAIP3, BCL10. lL8, GADD45B, NFKBIE and MIRN155, or into cluster CTA if at least two genes are overexpressed selected from the group consisting of HIST1H2BG, GINS1, CDC2, MAGEA3, BIRC5, MAGEA6, MAGEA12, PAGE1, PTTG1 and GAGE, or into cluster PRL3 if at least two genes are overexpressed selected from the group consisting of CCND2, PRL3, PTPRZ1, SOCS3 and SMYD3.

2. Method according to claim 1 wherein the sample comprises plasma cells.

3. Method according to claims 1 or 2, wherein the biological sample comprises plasma cells selected for CD138 expression.
